Europäisches Patentamt

European Patent Office

Office européen des brevets

(19)

(11) Veröffentlichungsnummer : **0 437 225 B1**

(12) # EUROPÄISCHE PATENTSCHRIFT

(45) Veröffentlichungstag der Patentschrift :
**20.04.94 Patentblatt 94/16**

(51) Int. Cl.⁵ : **A61K 47/02, A61K 47/18,
A61K 47/00, A61K 31/59**

(21) Anmeldenummer : **91100142.8**

(22) Anmeldetag : **04.01.91**

(54) **Topische Präparate.**

(30) Priorität : **10.01.90 US 463102**

(43) Veröffentlichungstag der Anmeldung :
**17.07.91 Patentblatt 91/29**

(45) Bekanntmachung des Hinweises auf die
Patenterteilung :
**20.04.94 Patentblatt 94/16**

(84) Benannte Vertragsstaaten :
**AT BE CH DE DK FR GB IT LI LU NL SE**

(56) Entgegenhaltungen :
**EP-A- 0 177 920
US-A- 4 855 294
STN INTERNATIONAL INFORMATION SERVI-
CES, DATENBANK: CHEMICAL ABSTRACTS,
Band 112, Nr. 12, Zusammenfassung Nr.
104869n, Columbus, Ohio, US**

(73) Patentinhaber : **F. HOFFMANN-LA ROCHE AG
Postfach 3255
CH-4002 Basel (CH)**

(72) Erfinder : **Kundu, Subhas Chandra
504 Curtis Road
Landing, N.J. 07850 (US)**
Erfinder : **Meltzer, Noel Mark
26 Blackburne Terrace
West Orange, N.J. 07052 (US)**

(74) Vertreter : **Grossner, Lutz, Dr. et al
Grenzacher Strasse 124 Postfach 3255
CH-4002 Basel (CH)**

**Beschreibung**

Die vorliegende Erfindung betrifft topische pharmazeutische Präparate mit 1α,25-Dihydroxycholecalciferol und einem geeigneten topischen pharmazeutischen Trägermaterial in Form von Emulsionen, die ein pH von etwa 6,5 bis 7,5 aufweisen. Die erfindungsgemässen Präparate besitzen sowohl bei Raumtemperatur als auch bei erhöhter Temperatur eine ausgezeichnete Stabilität.

Früher gab es bei topischen pharmazeutischen Präparaten mit 1α,25-Dihydroxycholecalciferol Stabilitätsprobleme infolge der Empfindlichkeit des 1α,25-Dihydroxycholecalciferols. Es wurde nun überraschend gefunden, dass die Stabilität topischer pharmazeutischer Präparate in Emulsionsform mit 1α,25-Dihydroxycholecalciferol verbessert werden kann, wenn das pH der wässrigen Phase so eingestellt wird, dass der pH-Bereich des topischen Präparates etwa 6,5 bis 7,5, vorzugsweise pH 6,8 bis 7,2 beträgt. Besonders bevorzugt ist ein pH-Wert des topischen Präparates von etwa 7,0.

Der hier verwendete Ausdruck Emulsion umfasst Oel-in-Wasser-Emulsionen, Wasser-in-Oel-Emulsionen und multiple Emulsionen.

Der hier verwendete Ausdruck topischer pharmazeutischer Träger bezieht sich auf ein Gemisch eines Viskositätserhöhers, eines lipophilen Solubilisierungsmittels, eines hydrophilen Solubilisierungsmittels, eines Emulgators, eines Emolliens, eines Konservierungsmittels, eines Antioxidans, eines Chelatisierungsmittels und eines Befeuchtungsmittels.

Die erfindungsgemässen Präparate umfassen Hautcrèmes, Tagescrèmes, Gele und Lotionen.

Tagescrèmes können Oel-in-Wasser-Emulsionen, Wasser-in-Oel-Emulsionen oder multiple Emulsionen sein. Tagescrèmes sind entweder Oel-in-Wasser-oder Wasser-in-Oel-Emulsionen. Lotionen sind üblicherweise Oel-in-Wasser-Emulsionen.

Die bevorzugten erfindungsgemässen Präparate sind ästhetisch elegante Oel-in-Wasser Tagescrèmes und Wasser-in-Oel Hautcrèmes.

Das pH der erfindungsgemässen Komposition liegt im Bereich von etwa 6,5 bis etwa 7,5, vorzugsweise etwa 6,8 bis 7,2. Der bevorzugte pH-Bereich der Präparate ist 7,0. Um dieses pH zu erreichen, wird die wässrige Phase der erfindungsgemässen Komposition eingestellt, vorzugsweise gepuffert auf einen pH von etwa 7,0 bis 8,0. Auf diese Weise resultiert ein pH des Gesamtpräparates im Bereich von etwa 6,5 bis 7,5, vorzugsweise 7,0.

Der hier verwendete Ausdruck "Gew..-%" bezieht sich auf Prozentangaben in Gewichtsteilen. 10 Gewichtsprozent bedeutet 10 g Inhaltsstoff auf 100 g der Gesamtkomposition.

Es ist bekannt, dass topische pharmazeutische Präparate mit 1α,25-Dihydroxycholecalciferol zur Behandlung von Hauterkrankungen wie Psoriasis verwendet werden können. Hierzu kann auf die US Patentschrift 4,610,978 verwiesen werden.

Die erfindungsgemässen topischen pharmazeutischen Präparate enthalten 1α,25-Dihydroxycholecalciferol zusammen mit topischen pharmazeutischen Trägermaterialien.

Insbesondere betrifft die Erfindung ästhetisch elegante Tagescrèmes, die gepufferte Oel-in-Wasser Emulsionen darstellen und 1α,25-Dihydroxycholecalciferol, einen Viskositätserhöher, ein lipophiles Solubilisierungsmittel, ein hydrophiles Solubilisierungsmittel, einen Emulgator, ein Emolliens, ein Konservierungsmittel, ein Antioxidans, ein Chelatisierungsmittel, ein Befeuchtungsmittel und einen Puffer enthalten.

Die erfindungsgemässen topischen pharmazeutischen Präparate können auch weitere Zusatzstoffe enthalten, die in topischen pharmazeutischen Präparaten verwendet werden. Solche Zusatzstoffe sind beispielsweise Riechstoffe und Substantivitätsmittel wie Polymere. Mit Substantivitätsmittel werden Stoffe bezeichnet, die einen verlängerten Kontakt zwischem dem pharmazeutischen topischen Präparat und der Haut bewirken.

Das Viskositätsmittel kann ein Alkohol, wie Cetylalkohol, Stearylalkohol, Cetostearylalkohol und Myristylalkohol sein. Der Viskositätserhöher kann auch ein Xanthangummi, ein Magnesiumaluminiumsilicat wie Veegum, Carbomer, Glycerylstearat, hydriertes Rizinusöl, Cetylpalmitat oder Stearinsäure sein, eine Kombination eines synthetischen, eines halbsynthetischen Wachses, eine Kombination von Glycerylstearat, Cetearylalkohol, Cetylpalmitat und ein Cocoglycerid-Gemisch sein oder eine Kombination von Glycerylhydroxystearat, Cetylpalmitat und einem Trihydroxystearin-Gemisch. Das Viskositätsmittel kann auch aus zwei oder mehreren der oben erwähnten Viskositätsmittel bestehen. Vorzugsweise ist es eine Kombination von Cetyl- und Stearylalkohol.

Das hydrophile Solubilisierungsmittel kann Dimethylisosorbid oder Transcutol sein oder ein aliphatischer Alkohol, wie Aethylalkohol, Isopropylalkohol, Polyäthylenglykol und insbesondere Propylenglykol.

Das lipophile Solubilisierungsmittel kann Rizinusöl sein, Isopropylmyristat, ein Alkohol wie Octyldodecanol, Isocetylalkohol, Oleylalkohol, Oleylcetylalkohol oder Triglyceride mit vegetabilen Fettsäuren mittlerer Kettenlänge wie Miglyole® (Gemische von Capryl- und Caprinsäuretriglyceriden, Propylenglykoldicaprylat und Dicaparat oder Gemische von Capryl- und Caprinsäuretriglyceriden) oder Neobee M-5. Das lipophile Solubilisierungsmittel ist vorzugsweise Mineralöl.

Der Emulgator kann ein Polysorbat sein, wie Polysorbat 20, Polysorbat 40, Polysorbat 60 und Polysorbat 80, ein Sorbitan, wie Sorbitanlaurat, Sorbitanoleat, Sorbitanpalmitat, Sorbitanstearat, Sorbitantrioleat oder Sorbitantristearat. Der Emulgator kann auch ein Glycerylmonostearat, ein Polyoxyäthylenstearat, ein Polyoxyäthylenlauryläther, PEG-20-glycerylstearat, Ceteareth-12, Ceteareth-20, Ceteareth-30, PPG-2-Ceteareth-9, Polyäthylenglykoläther und Oleylalkohol wie Oleth-5, Oleth-10, ein Gemisch von Oleth-5 und Oleth-10, Sterin wie Sojasterin, PEG-5-Sojasterin, PEG-10-Sterin, PEG-10-Stearin, PEG-16-Sojasterin, PEG-25-Sojasterin oder auch Natriumcetearylsulfat oder PEG-40 hydriertes Rizinusöl sein. Vorzugsweise ist der Emulgator ein Gemisch von Span 60 (Sorbitanmonostearat), Arlacel 165 (Glycerylmonostearat), und ein Polyoxyäthylenstearat-Gemisch und Tween 60 (Polysorbat 60).

Das Emolliens kann ein Ester sein, wie Olelyloleat, Octylstearat, Myreth-3-myristat, Hexyllaurat, Dibutyladipat, Isocetylstearat, Octyldodecylstearat, PEG-7-glycerylcocoat, Oleylerucat, ein Gemisch von Coco-caprylat und -caprat, Myristylmyristat, Cetearylisononanat, Decyloleat, ein Gemisch von Capryl- und Caprintriglyceriden, PEG-5-Laureth-5, Trihydroxymethoxystearin und ein Gemisch von Propylenglykol-dicaprylat und -dicaprat sein. Das Emolliens auch Rizinusöl oder Dioctylcyclohexan oder Mineralöl sein, vorzugsweise ist es Mineralöl.

Das Konservierungsmittel kann cis-1-(3-Chloräthyl)-3,5,7-triaza-1-azoniaadamantanchlorid, Sorbinsäure, Kaliumsorbat, Benzylalkohol, Benzalkoniumchlorid, Dichlorbenzylalkohol, N-(Hydroxymethyl)-N-(1,3-dihydroxymethyl-2,5-dioxo-4-imidazolidinyl)-N-hydroxymethyl)harnstoff, Borsäure, Chlorbutanol, Monothioglycerol, Methylparaben, Propylparaben sein oder vorzugsweise ein Gemisch von Methyl- und Propyl-paraben.

Das Antioxidans kann Propylgallat, butyliertes Hydroxytoluol, Ascorbylpalmitat, Natriumascorbat oder $\alpha$-Tocopherol sein, vorzugsweise ist es butyliertes Hydroxyanisol.

Das Chelatisierungsmittel kann Dimercaprol sein, Ascorbinsäure, Diphenylthiocarbazon, Aethylendiamintetraessigsäure (EDTA) oder vorzugsweise Dinatrium-EDTA.

Das Befeuchtungsmittel kann Glycerin, Elastin, Hyaluronsäure oder vorzugsweise Sorbit sein.

Die topischen pharmazeutischen Trägerstoffe, die erfindungsgemäss verwendet werden, sind schwach sauer. Um ein topisches pharmazeutisches Präparat im gewünschten pH-Bereich von etwa 6,5 bis 7,5 zu erhalten, ist es somit erforderlich, dass die wässrige Phase auf dieses pH eingestellt wird. Geeignete Mittel zur Einstellung des pH auf den gewünschten Bereich sind Natriumhydroxid oder Kaliumhydroxid oder Puffer, z.B. ein Gemisch von Natriumcitrat und Zitronensäure, ein Gemisch von Tris(hydroxymethyl)aminomethan und Tris(hydroxymethyl)aminomethan-hydrochlorid, oder Dinatriumphosphat. Andere Puffer, die mit topischen pharmazeutischen Präparaten verträglich sind, können auch verwendet werden. Der bevorzugte Puffer ist Dinatriumphosphat. Die Bestimmung des pH-Werts für die wässrige Phase, die dann mit dem jeweiligen pharmazeutischen Trägermaterial gemischt wird, sodass letztlich ein pH von etwa 6,5 bis 7,5 erreicht wird, liegt im Bereich des Fachwissens. Wenn das pH der wässrigen Phase im Bereich von etwa 7,0 bis 8,0 ist, resultiert gewöhnlich ein topisches pharmazeutisches Präparat, dessen pH im Bereich von etwa 6,5 bis 7,5 liegt.

Zur Herstellung der erfindungsgemässen topischen pharmazeutischen Präparate wird 1$\alpha$,25-Dihydroxycholecalciferol in der Oelphase gelöst. Die Oelphase und die wässrige Phase werden dann zur gewünschten Emulsion vermischt.

Die topischen pharmazeutischen Präparate der vorliegenden Erfindung enthalten etwa 0,00001 Gew.% bis etwa 0,1 Gew.%, vorzugsweise 0,0005 bis etwa 0,005 Gew.% 1$\alpha$,25-Dihydroxycholecalciferol. Die erfindungsgemässen topischen pharmazeutischen Präparate, die Oel-in-Wasser Emulsionen darstellen, können durch Einarbeiten von 1$\alpha$,25-Dihydroxycholecalciferol in Trägerformulierungen in folgenden Verfahrensstufen hergestellt werden.

Stufe 1: 1$\alpha$,25-Dihydroxycholecalciferol wird in einem hydrophilen Solubilisierungsmittel wie Propylenglykol unter Stickstoff gelöst. Vorzugsweise wird die erhaltene Lösung bei Raumtemperatur aufbewahrt, bis sie in Stufe 4 unten verwendet wird, um einen Abbau des 1$\alpha$,25-Dihydroxycholecalciferols zu vermeiden.

Stufe 2: Alle hydrophilen Bestandteile werden ausgewogen und dem Wasser zugesetzt. Das pH der Mischung wird auf einen Bereich von etwa 7,0 bis 8,0 eingestellt. Das erhaltene Gemisch wird unter Rühren auf eine maximale Temperatur von etwa 70-75°C erwärmt. Die erhaltene Lösung ist die wässrige Phase der letztlich hergestellten Emulsion.

Stufe 3: Alle lipophilen Bestandteile werden miteinander vermischt und unter Rühren auf eine maximale Temperatur von etwa 70-75°C erwärmt. Die erhaltene Lösung ist die Oelphase der letztlich hergestellten Emulsion.

Stufe 4: Die Lösung von Stufe 1 wird unter Stickstoff zur Oelphase von Stufe 3 unter Rühren gegeben, wobei die Temperatur bei etwa 70-75°C gehalten wurde. Der die Lösung von Stufe 1 enthaltende Behälter wird mit zusätzlichem Propylenglykol gewaschen und diese Waschlösung zum Gemisch von Stufe 4 gegeben.

Stufe 5: Die Oelphase von Stufe 4 wird unter Stickstoff zur wässrigen Phase von Stufe 2 gegeben, wobei die erhaltene Emulsion mit einem Homogenisator dispergiert wird. Die Homogenisierung wird fortgesetzt bis die Tröpfchengrösse der Emulsion etwa 1-20 Micron beträgt. Danach wird die Homogenisierung beendet und die Emulsion unter leichtem Rühren auf Raumtemperatur abkühlen gelassen.

Die obige Prozedur zur Herstellung der erfindungsgemässen topischen pharmazeutischen Präparate ist in den nachfolgenden Beispielen weiter erläutert.

Die erfindungsgemässen topischen pharmazeutischen Präparate werden durch Auftragen einer wirksamen Menge auf die zu behandelnde Fläche angewandt, beispielsweise durch Einreiben oder einfaches Auftragen des Präparates auf die psoriatische Haut.

Die nachfolgenden Beispiele erläutern die Erfindung weiter. $1\alpha,25\text{-}(OH)_2D_3$ bedeutet $1\alpha,25$-Dihydroxycholecalciferol.

Beispiel 1

Oel-in-Wasser Crème

| Inhaltsstoff | Gewichtsprozent |
|---|---|
| $1\alpha,25(OH)_2D_3$ | 0,00001 - 0,1 |
| Langkettiger Alkohol (Viskositätserhöher) | 0,1 - 20,0 |
| Lipophiles Solubilisierungsmittel | 0,2 - 40,0 |
| Hydrophiles Solubilisierungsmittel | 0,2 - 40,0 |
| Emulgator | 0,5 - 30,0 |
| Emolliens | 1,0 - 20,0 |
| Konservierungsmittel | 0,001 - 10,0 |
| Antioxidans | 0,001 - 5,0 |
| Chelatisierungsmittel | 0,001 - 5,0 |
| Befeuchtungsmittel | 0,1 - 10,0 |
| Wasser | 50 - 95,0 |
| Puffer | 2,0 - 12,0 |
| pH | 6,5 - 7,5 |

Beispiel 2

Oel-in-Wasser Crème

| | |
|---|---|
| $1\alpha,25(OH)_2D_3$ | 0,00004 - 0,1 |
| Cetylalkohol | 1,5 |
| Stearylalkohol | 2,5 |
| Sorbitanmonostearat | 2,0 |
| Mineralöl | 4,0 |
| Gemisch von Glycerylmonostearat und Polyoxyäthylenglykolstearat | 4,0 |
| Polysorbat 60 | 1,0 |
| Propylenglykol | 5,0 |
| Propylparaben | 0,05 |
| Butyliertes Hydroxyanisol | 0,05 |
| Sorbitlösung | 2,0 |
| Dinatrium-EDTA | 0,01 |
| Methylparaben | 0,18 |
| Wasser q.s. | 100,0 |
| pH eingestellt mit Natriumhydroxidlösung | 6,5 - 7,5 |

Die erfindungsgemässen topischen pharmazeutischen Präparate werden vorzugsweise in Epoxyharz-beschichteten Aluminiumtuben verpackt, die mit einem Phenyloxyharz versiegelt sind. Solche Packungen halten Sauerstoff und Licht von der Komposition fern. Alternativ können die topisch pharmazeutischen Präparate der vorliegenden Erfindung auch in Porzellan-Salbentöpfchen verpackt werden.

Beispiel 3

Oel-in-Wasser Crème

| Inhaltsstoff | Gew.-% | Variations-breite (Gew.-%) | Typischer Ansatz [g] |
|---|---|---|---|
| $1\alpha,25(OH)_2D_3$ | 0,00055* | - | 0,033 |
| gereinigtes Wasser | 80,61945** | 70-90 | 4837,167 |
| Methylparaben | 0,18 | 0,14-0,22 | 10,8 |
| Propylparaben | 0,05 | 0,04-0,06 | 3,8 |

| | | | |
|---|---|---|---|
| Sorbitlösung | 2,00 | 1-3 | 120,0 |
| Dinatrium-EDTA | 0,10 | 0,075-0,125 | 6,0 |
| Cetylalkohol | 1,50 | 0,5-2,5 | 90,0 |
| Stearylalkohol | 2,50 | 1,5-3,5 | 150,0 |
| Arlacel 165 (Gemisch von Glycerylmonostearat und Polyoxy-äthylenstearat | 4,00 | 3-5 | 240,0 |
| Sorbitanmonostearat | 2,00 | 1-3 | 120,0 |
| Polysorbat 60 (Tween 60) | 1,00 | 0,5-1,5 | 60,0 |
| Mineralöl, leicht | 4,00 | 3-5 | 240,0 |
| Propylenglykol | 5,00 | 4-6 | 300,0 |
| butyliertes Hydroxyanisol | 0,05 | 0,03-0,07 | 3,0 |
| Total | | | 6180,0 g |

--------------------------------

*    einschliesslich 10% Ueberschuss

**    einschliesslich 3% Ueberschuss, um Wasserverlust während des Herstellungsverfahrens zu kompensieren

Die wässrige Phase wurde mit Natriumhydroxidlösung auf pH 7,5 bis 8,0 eingestellt.

<u>Herstellungsverfahren</u>

1. Cetylalkohol, Stearylalkohol, Sorbitanmonostearat, Glycerylmonostearat und Polyoxyäthylenstearatgemisch, Polysorbat 60, Mineralöl und ein Teil Propylenglykol wurden in einem geeigneten Behälter gemischt und im Wasserbad geschmolzen.

2. Butyliertes Hydroxyanisol und Propylparaben wurden zum Material von Stufe 1 gegeben, gelöst und das Gemisch bei 70-75°C gehalten.

3. Dinatrium-EDTA und Methylparaben wurden in einer vorgeheizten Lösung von Sorbitlösung und gereinigtem Wasser gelöst.

4. $1\alpha,25(OH)_2D_3$ wurde in dem restlichen Propylenglykol gelöst und zum Material von Stufe 2 gegeben. Diese Operation, wie auch alle folgenden Operationen, wurden unter Stickstoff ausgeführt.

5. Das pH der Lösung von Stufe 3 wurde auf etwa 7,5 bis 8,0 eingestellt. Die Oelphase von Stufe 4 wurde zur wässrigen Phase von Stufe 3 gegeben, wobei mit einem Hochleistungs-Schermischer emulgiert wurde.

6. Das Produkt wurde auf Raumtemperatur abkühlen gelassen.

Beispiel 4

Gepufferte Oel-in-Wasser Crème

| Inhaltsstoffe | Gewichtsprozent |
|---|---|
| $1\alpha 25(OH)_2D_3$ | 0,0005 |
| Cetylalkohol | 1,5 |
| Stearylalkohol | 2,5 |
| Sorbitanmonostearat (Span 60) | 2,0 |
| Mineralöl | 4,0 |
| Arlacel 165 (Gemisch von Glycerylmonostearat und Polyoxyäthylenglykolstearat | 4,0 |
| Polysorbat 60 (Tween 60) | 1,0 |
| Propylenglykol | 5,0 |
| Propylparaben | 0,05 |
| butyliertes Hydroxyanisol | 0,05 |
| Sorbitlösung | 2,0 |
| Dinatrium-EDTA | 0,01 |
| Methylparaben | 0,18 |
| Wasser q.s. | 100,0 |
| pH eingestellt mit Dinatriumphosphat | 7,0 |

Herstellungsverfahren

1. Cetylalkohol, Stearylalkohol, Sorbitanmonostearat, Glycerylmonostearat und Polyoxyäthylenstearat-Gemisch, Polysorbat 60, Mineralöl und ein Teil Propylenglykol wurden im Wasserbad geschmolzen.
2. Butyliertes Hydroxyanisol und Propylparaben wurden im Material von Stufe 1 zugesetzt, gelöst und bei 70-75°C gehalten.
3. Dinatrium-EDTA und Methylparaben werden in einer vorgeheizten Lösung von Sorbitlösung und Wasser gelöst.
4. $1\alpha,25(OH)_2D_3$ wird im restlichen Propylenglykol gelöst und zum Material von Stufe 2 gegeben, wobei hier und in den folgenden Stufen unter Stickstoff gearbeitet wird.
5. Das pH der Lösung von Stufe 3 wird auf etwa 7,5 bis 8,0 eingestellt. Die Oelphase von Stufe 4 wird zur wässrigen Phase von Stufe 3 gegeben und in einem Hochleistungs-Schermischer emulgiert.
6. Das Produkt wird auf Raumtemperatur abgekühlt.

Beispiel 4a

Gepufferte Oel-in-Wasser Crème

| Inhaltsstoffe | Gewichtsprozent |
|---|---|
| $1\alpha,25(OH)_2D_3$ | 0,005 |
| Cetylalkohol | 1,5 |
| Stearylalkohol | 2,5 |
| Sorbitanmonostearat (Span 60) | 2,0 |
| Mineralöl | 4,0 |
| Arlacel 165 (Glycerylmonostearat und Polyoxyäthylenglykolstearat-Gemisch) | 4,0 |
| Polysorbat 60 (Tween 60) | 1,0 |
| Propylenglykol | 5,0 |
| Propylparaben | 0,05 |
| butyliertes Hydroxyanisol | 0,05 |
| Sorbitlösung | 2,0 |
| Dinatrium-EDTA | 0,01 |
| Methylparaben | 0,18 |
| Wasser q.s. | 100,0 |
| pH mit Dinatriumphosphat eingestellt auf pH | 7,0 |

Die obige Crème kann nach dem Verfahren von Beispiel 4 hergestellt werden.

Beispiel 5

Gepufferte Oel-in-Wasser Crème

| Inhaltstoff | Gewichtsprozent |
|---|---|
| $1\alpha,25(OH)_2D_3$ | 0,0005 |
| Cetylalkohol | 1,5 |
| Stearylalkohol | 2,5 |
| Sorbitanmonostearat (Span 60) | 2,0 |
| Mineralöl | 4,0 |
| Arlacel 165 (Glycerylmonostearat und Polyoxyäthylenglykolstearat-Gemisch) | 4,0 |
| Polysorbat 60 (Tween 60) | 1,0 |
| Propylenglykol | 5,0 |
| Propylparaben | 0,05 |
| butyliertes Hydroxyanisol | 0,05 |
| Sorbitlösung | 2,0 |
| Dinatrium-EDTA | 0,01 |
| Methylparaben | 0,18 |
| Wasser q.s. | 100,0 |
| pH mit Tris(hydroxymethyl)aminomethan und Tris(hydroxymethyl)aminomethan-hydrochlorid eingestellt auf | 7,4 |

Die obige Crème kann nach dem Verfahren von Beispiel 4 hergestellt werden.

Beispiel 6

Gepufferte Oel-in-Wasser Crème

| Inhaltstoff | Gewichtsprozent |
|---|---|
| $1\alpha,25(OH)_2D_3$ | 0,0005 |
| Cetylalkohol | 1,5 |
| Stearylalkohol | 2,5 |
| Sorbitanmonostearat (Span 60) | 2,0 |
| Mineralöl | 4,0 |
| Arlacel 165 (Glycerylmonostearat und Polyoxyäthylenglykolstearat-Gemisch) | 4,0 |

| | |
|---|---|
| Polysorbat 60 (Tween 60) | 1,0 |
| Propylenglykol | 5,0 |
| Propylparaben | 0,05 |
| butyliertes Hydroxyanisol | 0,05 |
| Sorbitlösung | 2,0 |
| Dinatrium-EDTA | 0,01 |
| Methylparaben | 0,18 |
| Wasser q.s. | 100,0 |
| pH eingestellt mit Natriumhydroxidlösung auf | 6,6 |

Die obige Crème kann nach dem Verfahren von Beispiel 4 hergestellt werden.

Beispiel 7

Gepufferte Oel-in-Wasser Crème

| Inhaltstoff | Gewichtsprozent |
|---|---|
| $1\alpha,25(OH)_2D_3$ | 0,005 |
| Cetylalkohol | 1,5 |
| Stearylalkohol | 2,5 |
| Sorbitanmonostearat (Span 60) | 2,0 |
| Mineralöl | 4,0 |
| Arlacel 165 (Glycerylmonostearat und Polyoxyäthylenglykolstearat-Gemisch) | 4,0 |
| Polysorbat 60 (Tween 60) | 1,0 |
| Propylenglykol | 5,0 |
| Propylparaben | 0,05 |
| butyliertes Hydroxyanisol | 0,05 |
| Sorbitlösung | 2,0 |
| Dinatrium-EDTA | 0,01 |
| Methylparaben | 0,18 |
| Wasser q.s. | 100,0 |
| pH eingestellt mit Natriumhydroxidlösung auf | 7,1 |

Die obige Crème kann nach dem Verfahren von Beispiel 4 hergestellt werden.

Beispiel 8

Wasser-in-Oel Crème

| Inhaltsstoff | Gew.-% | Variations-breite (Gew.-%) | Funktion |
|---|---|---|---|
| $1\alpha,25(OH)_2D_3$ | 0,00055-0,0055 | 0,00001-0,1 | Wirkstoff |
| Hydroxyliertes Lanolin | 2,5 | 1,75-3,25 | Emulgator |
| Mineralöl und Lanolinalkohol | 16,5 | 11,55-21,45 | Emulgator |
| Mineralöl, leicht | 20,0 | 14,0-26,0 | Emolliens |
| mikrokristallines Wachs, Smp. 170-175°C | 16,0 | 11,2-20,8 | Verdickungs-mittel |
| Propylparaben | 0,05 | 0,04-0,06 | Konservie-rungsmittel |
| Propylenglykol | 5,00 | 4,0-6,0 | Solubilisator |
| Methylparaben | 0,18 | 0,14-0,22 | Konservie-rungsmittel |
| Dinatrium-EDTA | 0,10 | 0,075-0,125 | Chelatisie-rungsmittel |
| butyliertes Hydroxyanisol | 0,05 | 0,03-0,07 | Antioxidans |
| Wasser | 39,62 | 28,0-52,0 | Vehikel |
| pH | 6,5-7,0 | | |

Herstellungsverfahren:

1. Hydroxyliertes Lanolin, Mineralöl und Lanolinalkohol, leichtes Mineralöl, mikrokristallines Wachs und ein Teil Propylenglykol werden im Wasserbad bei 70-75°C zusammengeschmolzen.
2. Das butylierte Hydroxyanisol und Propylparaben werden dem Gemisch von Stufe 1 zugesetzt, gelöst und bei 70-75°C gehalten.
3. Dinatrium-EDTA und Methylparaben werden im vorgeheizten Wasser gelöst.
4. $1\alpha,25(OH)_2D_3$ wird in dem restlichen Propylenglykol gelöst und zum Material von Stufe 2 gegeben, wobei diese und die folgenden Operationen unter Stickstoff ausgeführt werden.
5. Das pH wird auf 7,5 bis 8,0 mit Dinatriumphosphat eingestellt. Danach wird das Oel von Phase 4 zur wässrigen Phase von Stufe 3 unter Emulgieren mit einem Hochleistungs-Schermischer gegeben.
6. Das Produkt wird auf Raumtemperatur abgekühlt.

Beispiel 9

Wasser-in-Oel Crème

| Inhaltsstoff | Gew.-% | Variations-breite (Gew.-%) | Funktion |
|---|---|---|---|
| $1\alpha,25(OH)_2D_3$ | 0,00055-0,0055 | 0,00001-0,1 | Wirkstoff |
| Petrolatum, Lanolin und Lanolinalkohol | 7,0 | 4,9-9,1 | Emulgator |
| Mineralöl, leicht | 12,0 | 8,4-15,6 | Emolliens |
| mikrokristallines Wachs, Smp. 170-175°C | 9,0 | 6,3-11,7 | Verdickungs-mittel |
| Sorbitan Sesquioleat | 2,0 | 1,4-2,6 | Emulgator |
| Propylparaben | 0,05 | 0,04-0,06 | Konservie-rungsmittel |
| butyliertes Hydroxyanisol | 0,05 | 0,04-0,06 | Solubilisie-rungsmittel |
| Propylenglykol | 5,00 | 4,0-6,0 | Solubilisie-rungsmittel |
| Methylparaben | 0,18 | 0,14-0,22 | Konservie-rungsmittel |
| Dinatrium-EDTA | 0,10 | 0,075-0,125 | Chelatisie-rungsmittel |
| Wasser | 64,62 | 45,0-84,0 | Vehikel |
| pH | 6,5-7,0 | | |

Die Crème wird in der gleichen Weise wie die Crème von Beispiel 8 hergestellt. Der Puffer ist Dinatriumphosphat.

Die folgenden Daten geben einen Vergleich der Stabilität von $1\alpha,25$-Dihydroxycholecalciferol in ungepuffertem Oel-in-Wasser Vanishing Crèmes und in den erfindungsgemässen gepufferten Oel-in-Wasser Crèmes. Sie wurden durch Analysen von Crèmes erhalten, die in 15 g Epoxy-laminierten Aluminiumtuben mit weissen Polyäthylenverschlüssen aufbewahrt wurden.

Eine wie in Beispiel 3 hergestellte Crème mit 0,0005 Gew.-% Wirkstoff, jedoch ohne Puffer (pH 5,5-6,0) wurde 12 Monate bei 25°C aufbewahrt und zeigte einen Wirksamkeitsverlust von $1\alpha,25$-Dihydroxycholecalci-ferol von 11%. Im Gegensatz dazu zeigte die 0,0005 Gew.-%ige Crème von Beispiel 3 (pH 6,5) bei der Lagerung während 12 Monaten bei 25°C ein Wirksamkeitsverlust von $1\alpha,25$-Dihydroxycholecalciferol von nur 2%.

Eine wie in Beispiel 7 hergestellte Crème mit 0,005 Gew.-% Wirkstoff, jedoch ohne Puffer (pH 5,5-6,0) wurde 6 Monate bei 25°C aufbewahrt und zeigte einen Wirksamkeitsverlust von 7%, Im Gegensatz dazu zeigte die gemäss Beispiel 7 hergestellte 0,005 Gew.-%ige Crème (pH 7,1) bei gleichen Lagerungsbedingungen keinen Wirksamkeitsverlust.

Eine wie in Beispiel 7 hergestellte, jedoch nicht gepufferte 0,005 Gew.-%ige Crème (pH 5,5-6,0) wurde 6 Monate bei 37°C aufbewahrt und zeigte einen Wirksamkeitsverlust von 18%. Im Gegensatz dazu zeigte die Crème von Beispiel 7 (pH 7,1) bei den gleichen Lagerungsbedingungen einen Wirksamkeitsverlust von 4%. Die obigen Daten zeigen, dass die erfindungsgemässen Präparate stabiler als die Präparate des Standes der Technik sind.

**Patentansprüche**

1. Topisches pharmazeutisches Präparat in Emulsionsform, das etwa 0,00001 bis etwa 0,1 Gew.-% 1α,25-Dihydroxycholecalciferol und pharmazeutische Trägermaterialien enthält und dessen pH etwa 6,5 - 7,5 beträgt.

2. Präparat nach Anspruch 1, wobei der pH etwa 6,8 - 7,2 beträgt.

3. Präparat nach Anspruch 1, wobei der pH etwa 7,0 beträgt.

4. Präparat nach den Ansprüchen 1-3, wobei der pH mittels NaOH, KOH, $Na_2HPO_4$ oder Tris(hydroxymethyl)aminomethan und Tris(hydroxymethyl)aminomethen-hydrochlorid eingestellt ist.

5. Präparat nach den Ansprüchen 1-3, wobei der Gehalt an 1α,25-Dihydroxycholecalciferol etwa 0,0005 bis 0,005 Gew.-% beträgt.

6. Präparat nach den Ansprüchen 1-5 in Form eines Gels, einer Lotion oder einer Tagescreme.

7. Präparat nach den Ansprüchen 1-5 in Form einer Tagescreme.


**Claims**

1. A topical pharmaceutical preparation in the form of an emulsion which contains about 0.00001 to about 0.1 wt.% of 1α,25-dihydroxycholecalciferol and pharmaceutical carrier materials and whose pH is about 6.5-7.5.

2. A preparation according to claim 1, wherein the pH is about 6.8-7.2.

3. A preparation according to claim 1, wherein the pH is about 7.0.

4. A preparation according to claims 1-3, wherein the pH is adjusted using NaOH, KOH, $Na_2HPO_4$ or tris(hydroxymethyl)aminomethane and tris(hydroxymethyl)aminomethane (sic) hydrochloride.

5. A preparation according to claims 1-3, wherein the content of 1α,25-dihydroxycholecalciferol is about 0.0005 to 0.005 wt.%.

6. A preparation according to claims 1-5 in the form of a gel, a lotion or a vanishing skin cream.

7. A preparation according to claims 1-5 in the form of a vanishing skin cream.


**Revendications**

1. Préparation pharmaceutique topique sous forme d'émulsion, qui comprend environ 0,00001 à environ 0,1 % en poids de 1α,25-dihydroxycholécalciférol et des matériaux véhicules pharmaceutiques et dont le pH est d'environ 6,5 - 7,5.

2. Préparation selon la revendication 1 dans laquelle le pH est d'environ 6,8 - 7,2.

3. Préparation selon la revendication 1 dans laquelle le pH est d'environ 7,0.

4. Préparation selon les revendications 1-3 dans laquelle le pH est ajusté au moyen de NaOH, KOH, $Na_2HPO_4$ ou tris(hydroxyméthyl)aminométhane et tris(hydroxyméthyl)aminométhane-chlorhydrate.

5. Préparation selon les revendications 1-3, dans laquelle la teneur en 1α,25 dihydroxycholécalciférol est d'environ 0,0005 à 0,005 % en poids.

6. Préparation selon les revendications 1-5 sous forme d'un gel, d'une lotion ou d'une crème de jour.

7. Préparation selon les revendications 1-5 sous forme d'une crème de jour.